# NEW EUROPEAN PATENT SPECIFICATION

(11) **EP 0 320 086 B2**
(45) Date of publication and mention of the opposition decision: **03.03.1999**
(45) Mention of the grant of the patent: 15.09.1993
(21) Application number: 88306711.8
(22) Date of filing: 21.07.1988
(51) Int. Cl.: G01N 21/64, G01N 33/18, G01N 31/22, C02F 1/66

(54) **Method for detection and quantitative analysis for water treatment chemicals**
Verfahren zum Nachweis und mengenmässiger Analyse von Wasserbehandlungschemikalien
Méthode pour la détection et l'analyse quantitative de produits chimiques de traitement de l'eau

(30) Priority: 11.12.1987 US 131761
(43) Date of publication of application: 14.06.1989
(73) Proprietor: UNILEVER N.V., 3013 AL Rotterdam (NL); UNILEVER PLC, London EC4P 4BQ (GB)
(72) Inventor: Baker, Gary L., Alexandria Kentucky (US); Christensen, Ronald J., Montgomery Ohio (US)
(74) Representative: Rosen Jacobson, Frans Lucas M.

(56) References cited:
- FR-A- 1 019 006
- GB-A- 2 143 638
- GB-A- 2 152 937
- US-A- 2 815 328
- US-A- 4 300 908
- US-A- 4 659 676
- US-A- 4 762 167
- US-A- 4 783 314
- Product Bulletin NALCO 8391 (1983)
- Product Bulletin NALCO 39-L including general description of the product (1983)
- Letter from Linear Films Inc. to Dubois Chemical Co., dated October 1, 1986
- Combined Declaration of Ronald Christensen and Gary Baker dated April 19, 1989
- Declaration of Ronald Christensen dated February 23, 1990
- Meyer and Stanbro, "Fluorescent Dye, A Novel Technique to Trace Cooling Tower Drift", 4th Joint Conference on sensing of environmental pollutants, 1977, pp. 618-623
- Smart and Laidlaw, "An Evaluation of Some Fluorescent Dyes for Water Tracing", Water Resources Research, Vol. 13, No. 1 (February 1977), pp. 15-33
- Brown et al., "The Use of Bromophenol Blue as a Tracer in Sewage Works", Water Res., Vol. 18, No. 9, pp. 1083-1087 (1984)
- Wimpenny, J.W.T., "Treatment of Industrial Effluents", edited by A.G. Calley, C.F. Forster and D.A. Stafford, New York, 1976, Chapter 21 entitled "Water Tracing", pp. 346-375
- Completed document of first page of D2
- Completed document of pages 2 and 3 of D2
- Roempps Chemie-Lexikon, 8. Auflage, pp. 1336, 2382, 3130, 3580, 3581, 3655
- Product Bulletin NALBLUE 8984 and trademark application of NALBLUE

## Description

There are many different types of circulating water systems. Three basic types are water cooling towers which are open systems, boilers which are closed systems, and chilled water systems which are also closed. More recently, cross-over cooling water systems, where both tower water and chilled water are combined for a period, are being employed.

If ultra high purity water were available in ample supply there would be little reason chemically to treat any of the water added to these systems. In actuality, however, highly pure water is rarely available. Therefore, operators of water systems must resort to chemical treatment to prevent damage to the water system which can be caused by the impurities contained in water.

There are literally dozens of treatment compositions that are currently added to these systems. The selection of the particular treatment agent depends on the type of water added, the particular water system employed, and operational conditions. Some of the types of treatment compositions added include dispersants, descalants, scale inhibitors, algacides, biocides, corrosion inhibitors, oxygen scavengers and pH modifiers. In each category of composition there are also a number of different particular compositions which can be used to accomplish the desired result. For example, phosphonates are typically used as anti-scaling agents and corrosion inhibitors. Chelating agents such as ethylene-diamine tetraacetic acid and nitrilotriacetic acid are also anti-scaling agents. Corrosion inhibitors also include aromatic azoles, alkaline earth metal molybdates and so on.

Water systems are treated by adding the selected desired treatment agents separately or more likely a combination of treatment agents is dispensed into the water in the water system. Combinations for example could include corrosion inhibitors in combination with descalants and biocides for a water cooling tower. A boiler treatment composition may for example include an anti-scalant, corrosion inhibitors and oxygen scavenger.

Whether the treatment compositions are added separately or as a mixture it is necessary to determine the concentration level of the treatment agent or agents in the water system. Typically this has been done by simply taking a sample of the water in the system and performing a series of chemical analysis such as pH and various titrations to determine the concentration of the chemicals added.

One chemical that has been added in the past has been tannins and a similar composition lignins. These are anionic compositions which have been used in the past as dispersants. They are no longer the preferred dispersants since certain water soluble polymers have been developed which by far surpass the dispersing activities of these chemicals. However, these chemicals did have one incidental advantage in that they had a color and could be visually detected within a water system. Thus the boiler operator could visually detect the concentration of tannin or lignin in the system. When used they were added separately to the system. Therefore they did not provide any indication of the concentration of any other component.

Because tannins and lignins are anionic they cannot be added to a mixture which includes cationic treatment agents such as other dispersants and the like which can be cationic. In concentrated solutions, the tannins and lignins would precipitate out of solution and be totally ineffective.

Accordingly, to determine the concentration of the treatment agents in a water system one must perform complex titrations and other tests. This is inefficient and can result in ineffective treatment or a waste of treatment agents.

As further prior art, known from around 1978 is a product designated by the trade mark NALBLUE, which is a composition for use in treating air washers and containing a dye to allow the user to visually determine a proper dosage level of a main treatment active.

In accordance with the present invention, which is as defined in claim 1 hereof, an inert water soluble dye is added and mixed with a concentrated treatment agent for a water system and is dispensed into the water system with the treatment agent. Since the amount of dye added to the water system is proportional to the amount of treatment agent added, the concentration of the treatment agent can be determined by measuring the concentration of the dye. Thus, the intensity of the colour of the water provides an indication of the concentration of the treatment agent.

Concentration of the treatment agent can then be determined by photometric and/or visual means and more specifically by a colorimetric method using visible light detectors. Fluorometric techniques may also be used. Selecting a dye with an absorbance which varies linearly with concentration at application concentration simplifies colorimetric analysis.

A photodegradable dye can be employed which when dispensed into the environment does not have a long lasting and noticeable effect on the environment and thus is environmentally acceptable. Surprisingly even in open systems such as water cooling towers where the water treatment agent is exposed to sunlight for a period of time a photodegradable dye can be employed without being totally inactivated when exposed to the light.

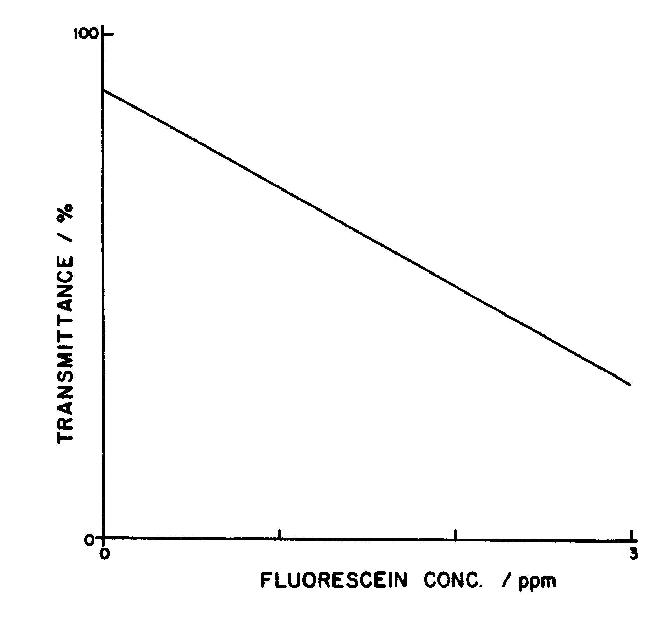

The invention will now be further described by way of example with reference to the accompanying drawing which is a graphical depiction of the percent transmittance of 500nm light relative to the concentration of a fluorescein dye.

Water treatment compositions used in the method of the invention comprise a water based concentrated composition which incorporates a known quantity of a water soluble dye as well as a known quantity of one or more water treating chemicals. By detecting the concentration of the dye in a circulating water system one can also detect the corresponding concentration of the active ingredients of the water treatment composition or determine the amount of treatment product added. Also, when slug doses of the treatment is initially added, system water volume can be determined.

Water circulating systems to be treated with such a composition may include water cooling towers, evaporative condensers, boilers, and chilled water systems. Each of these systems require one or more treating chemicals or agents added to the water in the systems. Treatment compositions may basically include any chemical which will be added to the water of a water cooling tower, boiler or chilled water system to alter any physical or chemical activity of the water. These chemical compositions include dispersants including organophosphorous compounds particularly organophosphorous carboxylic acids and the phosphonates. Typically the phosphonates include aminomethylene phosphonic acid and 1-hydroxylethyladene-1,1-diphosphonic acid. A commonly employed organic phosphorus carboxylic acid is 2-phosphonobutane-1,2,4-tricarboxylic acid.

These chemical compositions may also include corrosion inhibitors such as alkaline earth metal molybdates, chromates and nitrite salts which are used at higher concentrations. Sodium molybdate is an anodic inhibitor and is the most commonly used molybdate inhibitor. Other corrosion inhibitors include aromatic azoles primarily used as corrosion inhibitors for copper and its alloys. Generally included within the aromatic azoles are benzotriazole, tolyltriazole and mercaptobenzothiazole.

Biocides may also be employed. These may include ClO₂, chlorine, chlorine release compounds such as chlorinated isocyanurates. hypochlorites, and chlorinated hydantoins. Oxidizers such as chlorine, present in the cooling water system at concentrations greater than about 0.5 ppm, will degrade the fluorescein Hydrogen peroxide tends to cause precipitation of the fluorescein when added to the treated cooling water. Quaternary ammonium compounds are the primarily non-oxidizing biocides and biostats. These are cationic surface active chemicals which are most effective against algae and bacteria at alkaline pH.

Chelants may also be used as hardness sequestering agents. These include ethylenediaminetetraacetic acid (EDTA) and nitrilotriacetic acid (NTA).

Anionic polymers are widely used in industrial boilers for sludge conditioning. These are credited with inhibiting scale formation and with removing existing scale by several mechanisms. Such anionic polymers include polyacrylates, polymethacrylates. polymaleic anhydride and various copolymers of these. Synthetic sulfonated polymers, synthetic carboxylated polymers and carboxymethylcellulose are also used.

Oxygen scavengers are used primarily in boiler operations and chilled water systems. Oxygen scavengers include sodium sulfite, hydrazine and erythorbic acid and salts thereof. In boilers neutralizing amines are used to combat the interaction of carbon dioxide with steam which forms carbonic acid. The neutralizing amines may include cyclohexylamine. morpholine, and diethylaminoethanol. Filming amines are used to establish a continuous protective film over surfaces in the after boiler section. These would include octadecylamine.

There are also a variety of different chemicals used to adjust boiler water pH including sodium hydroxide and sodium carbonate. Other compositions may include sodium nitrate which inhibits caustic embrittlement, and anti-foaming agents like polyglycols, silicones and polyamides.

One or more of these chemical compositions or agents are added to a water system in an attempt to attain a desired use concentration generally defined in parts per million of the actives of the chemical compositions. These are however purchased and dispensed as concentrated aqueous solutions which are generally defined in terms of grams of actives per litre of concentrated treatment composition. Where the treatment composition is a mixture of treatment agents the concentration of each agent is proportioned to the desired use concentration.

Thus a known concentration Q₁ of an inert water soluble dye is added to the concentrated treatment composition. For use in the present invention the water soluble dye must be inert. For purposes of the present invention, 'inert' means that it must have neither appreciable anionic nor cationic characteristics in concentrated forms. It should also be thermally stable, and stable to both reducing and slightly oxidizing environments.

The dye should be photodetectable to provide advantageous benefits. By photodetectable it must be detected by light reflectance, transmittance or absorbance in the ultraviolet, infrared, or visible spectrums. Preferably, the dye will be fluorescent and detectable by one of these means using visible light. Under these circumstances the concentration can be initially visually detected and can subsequently be detected by means of a colorimeter which is quite inexpensive relative to ultraviolet and infrared spectrophotometers. Visual detection can be facilitated by the use of a color comparator.

Further, since most water systems periodically bleed off water in the system and dispense this into the environment, the dye should preferably be nonstaining and non-persistent. More particularly it should be a dye which degrades naturally and quickly in the environment and preferably would be a photodegradable dye. Suitable non-persistent dyes include fluorescein, Rhodamine B, Rhodamine WT and Lissamine. All of these are non-persistent and are currently used in water flow studies in the environment. A preferred dye is fluorescein which degrades very quickly in the environment.

The dye will be added to the concentrated treatment composition and dispensed with the treatment composition into the water system. The amount of dye added to the concentrated treatment composition will depend on the amount of treatment composition intended to be added to the water system to provide the desired concentration level of the treatment composition generally in terms of parts per million. It is preferable that the concentration of the dye present in the water system will be within a concentration range where the change in amount of dye will provide a linear response on the spectrophotometer. This is shown with respect to fluorescein in the drawing.

This figure shows the percent transmittance of light at 500nm versus the concentration of fluorescein at a concentration level of 0.3-3 parts per million. At this concentration range the change in concentration of fluorescein with respect to transmittance is basically linear making it very easy to determine photometrically the concentration of fluorescein in the water system.

Since the concentration of the fluorescein in the treatment agent is known and the concentration of the fluorescein in the water can be detected, the concentration of the water treatment agent can be determined. The optimum concentration of fluorescein is 1.2 ppm. Thus, where the desired concentration of treatment composition is 120 ppm, the treatment composition should be 1% fluorescein.

This of course will change depending on the dye used, the wave length of light at which this is measured and the optimum concentration of the particular dye.

The invention will be further appreciated in light of the following examples of concentrations of components that could be added to boilers, water cooling towers and chilled water systems. These provide typical desired optimum concentrations and typical desired concentrations of the fluorescein dye that would be added to these compositions.

| Cooling Water Treatment Formula | | |
|---|---|---|
| Order of Addition | Ingredient | Percent |
| 1 | Water | 87.6 |
| 2 | Phosphonate (HEDP - 60%) | 3.0 |
| 3 | Polyacrylic Acid - 50% | 1.0 |
| 5 | Tolytriazole - 50% | 2.0 |
| 6 | Flourescein | 0.4 |
| 4 | Sodium Hydroxide - 50% | 6.0 |
| Applied at 300-600 ppm conc. in the system. | | |

| Boiler Water Treatment Formula | |
|---|---|
| Ingredient | Percent |
| Water | 72.35 |
| Disodium Phosphate | 6.00 |
| Polyacrylic Acid - 50% | 1.50 |
| Sodium Sulfite | 5.00 |
| Potassium Hydroxide | 15.00 |
| Fluorescein | 0.15 |
| Maintained at 800 to 1200 ppm in the boiler. | |

These compositions are given merely by way of example and the particular chemical compositions that would be added or combinations of chemical compositions that would be added to the particular system will vary widely particularly depending on the make-up water added to the system which varies based on geographic location.

In carefully conducted field tests where a water treatment composition having .4% fluorescein was added to a water cooling tower, the accuracy of this method was compared to a standard industry test, the thorium nitrate test. By measuring make up water and the mass of treatment composition added, the concentration was determined to be 384 ppm. Using a colorimeter to detect the concentration of fluorescein, the concentration was measured at 341 ppm. Using color comparator, the concentration was determined to be 400 ppm. The thorium nitrate test, which tests for phosphonate concentration, indicated the concentration was 469 ppm. Thus, detecting concentration by detecting fluorescein concentration is substantially more accurate than the thorium nitrate test and much simpler to apply.

## Claims

1. A method of determining the concentration of a treatment composition present in the water in a water system, which method comprises adding into the water a mixture comprising a known amount of an inert water soluble dye and a known amount of the treatment composition, and determining the concentration of the treatment composition present in the water by determining the concentration of the inert dye in the treated water in the system, the concentration of the inert dye in the water being proportional to the concentration of the treatment composition which is present in the water.

2. A method as claimed in Claim 1 wherein the dye is a photodegradable dye.

3. A method as claimed in Claim 1 or 2 wherein the dye is fluorescein, Rhodamine B, Rhodamine WT or Lissamine.

4. A method as claimed in any of the preceding claims wherein the dye is fluorescein, maintained at concentration levels between 0.3 and 3.0 ppm.

5. A method as claimed in any of Claims 1 to 4 wherein the concentration of said dye is determined by photodetection.

6. A method as claimed in any of Claims 1 to 4 wherein the concentration of the dye is visually determined.

7. A method as claimed in any one of the preceding claims, wherein the water system is a water cooling tower, evaporative condenser, boiler or chilled water system.

## Patentansprüche

1. Verfahren zum Bestimmen der Konzentration einer im Wasser eines Wassersystems vorhandenen Behandlungszusammensetzung, bei dem man dem Wasser ein Gemisch zusetzt, wobei das Gemisch eine bekannte Menge eines inerten, wasserlöslichen Farbstoffs und eine bekannte Menge der Behandlungszusammensetzung enthält, und bei dem man die Konzentration der im Wasser vorhandenen Behandlungszusammensetzung durch Bestimmen der Konzentration des inerten Farbstoffs im behandelten Wasser des Systems bestimmt.

2. Verfahren nach Anspruch 1, wobei der Farbstoff ein photoabbaubarer Farbstoff ist.

3. Verfahren nach Anspruch 1 oder 2, wobei der Farbstoff Fluoreszein, Rhodamin B, Rhodamin WT oder Lissamin ist.

4. Verfahren nach einem der vorhergehenden Ansprüche, wobei der Farbstoff Fluoreszein ist, welches bei Konzentrationswerten zwischen 0,3 und 3,0 ppm gehalten wird.

5. Verfahren nach einem der Ansprüche 1 bis 4, wobei man die Konzentration des Farbstoffs durch Photobestimmung bestimmt.

6. Verfahren nach einem der Ansprüche 1 bis 4, wobei man die Konzentration des Farbstoffs visuell bestimmt.

7. Verfahren nach einem der vorhergehenden Ansprüche, wobei das Wassersystem ein Wasserkühlturm, ein Verdunstungsverflüssiger, ein Boiler oder ein Tiefkühlwassersystem ist.

## Revendications

1. Procédé de détermination de la concentration d'une composition de traitement présente dans l'eau dans un système d'eau, ce procédé comprenant l'addition d'un mélange à l'eau, le mélange comprenant une quantité connue d'un colorant soluble dans l'eau, inerte et une quantité connue de la composition de traitement, et la détermination de la concentration de la composition de traitement présente dans l'eau par détermination de la concentration du colorant inerte dans l'eau traitée présente dans le système, la concentration du colorant inerte dans l'eau étant proportionnelle à la concentration de la composition de traitement qui est présente dans l'eau.

2. Procédé selon la revendication 1, dans lequel le colorant est un colorant photodégradable.

3. Procédé selon les revendications 1 ou 2, dans lequel le colorant est la fluorescéine, la Rhodamine B, la Rhodamine WT ou la Lissamine.

4. Procédé selon l'une quelconque des revendications précédentes, dans lequel le colorant est la fluorescéine, maintenue à des niveaux de concentration compris entre 0,3 et 3,0 ppm.

5. Procédé selon l'une quelconque des revendications 1 à 4, dans lequel la concentration de ce colorant est déterminée par photodétection.

6. Procédé selon l'une quelconque des revendications 1 à 4, dans lequel la concentration du colorant est déterminée visuellement.

7. Procédé selon l'une quelconque des revendications précédentes, dans lequel le système d'eau est une tour de refroidissement par eau, un condenseur à évaporation, une chaudière ou un système à eau refroidie.
